# EUROPEAN PATENT APPLICATION

(11) **EP 4 621 053 A1**
(43) Date of publication of application: **24.09.2025**
(21) Application number: 22968130.9
(22) Date of filing: 14.12.2022
(51) Int. Cl.: C12N 15/10, C07K 1/26, C12M 1/00

(54) **NUCLEIC ACID SEQUENCING SAMPLE PRETREATMENT DEVICE AND METHOD**

(71) Applicant: BGI Shenzhen, Shenzhen, Guangdong 518083 (CN); BGI Shenzhen Co., Limited, Guangdong 518083 (CN)
(72) Inventor: ZHANG, Meng, Shenzhen, Guangdong 518083 (CN); ZHANG, Yuning, Shenzhen, Guangdong 518083 (CN); LI, Yuxiang, Shenzhen, Guangdong 518083 (CN); YUN, Quanxin, Shenzhen, Guangdong 518083 (CN); DONG, Yuliang, Shenzhen, Guangdong 518083 (CN); ZHANG, Wenwei, Shenzhen, Guangdong 518083 (CN)
(74) Representative: HGF
(86) International application number: PCT/CN2022/138867
(87) International publication number: WO 2024/124419

(57) **Abstract**

Provided are a sample pretreatment device and method for nucleic acid sequencing. The sample pretreatment device for nucleic acid sequencing includes a carrier, a first electrode, a second electrode, and a controller. The carrier is provided with a pretreatment chamber. The carrier is provided with a fluid inlet and a fluid outlet that communicate with the pretreatment chamber. The first electrode is disposed on the top wall of the pretreatment chamber. The second electrode is disposed on the bottom wall of the pretreatment chamber. The first electrode and the second electrode are electrically connected to the controller.

## Description

### TECHNICAL FIELD

The present application relates to the field of gene sequencing, for example, a sample pretreatment device and method for nucleic acid sequencing.

### BACKGROUND

Until now, nanopore sequencing technology has been widely applied in gene sequencing. When the nanopore sequencing technology is used for nucleic acid sequencing with electrophoresis, nucleic acids, uniformly distributed in a nucleic acid sample, can pass through nanopores under the action of an electric field, thereby generating electrical signals for analyzing nucleic acid characteristics.

The electrophoresis phenomenon refers to a process in which charged particles move toward the electrode of opposite charge under the action of an electric field. In the nucleic acid sample, a short nucleic acid fragment has a lower charge than a long nucleic acid fragment and thus has a higher electrophoretic mobility. In electrophoresis with a fixed endpoint, objects with higher electrophoretic mobility can reach the endpoint earlier and thus have higher electrophoretic priority. For objects of the identical type, those starting closer to the endpoint exhibit higher electrophoretic priority due to shorter migration distances. At a given initial position, objects with a higher electrophoretic mobility have a higher electrophoretic priority. If the nucleic acids are evenly distributed in the nucleic acid sample, the initial positions of the nucleic acids in electrophoresis can be considered identical, and thus the short nucleic acid fragments have the higher electrophoretic priority.

When using the nanopore sequencing technology to detect and analyze nucleic acid characteristics to achieve nucleic acid sequencing, the long nucleic acid fragments provide more valuable sequencing data than the short nucleic acid fragments, and the sequencing data obtained from the long nucleic acid fragments can yield more accurate sequencing results. Therefore, under fixed sequencing duration, sample utilization can be quantified by the proportion of long nucleic acid fragments that successfully translocate through the nanopores in electrophoresis and generate valid sequencing data. In the related art of nanopore sequencing and under electrophoresis, after using a pipette to load the evenly mixed sample into a chamber through a sample port, shorter nucleic acid fragments translocate through the nanopores more rapidly, thus the shorter nucleic acid fragments exhibit higher electrophoretic priority and better utilization in sequencing than longer nucleic acid fragments. This bias leads to insufficient sample utilization, relatively low accuracy of the sequencing, and degraded sequencing quality.

### SUMMARY

The present application provides a sample pretreatment device for nucleic acid sequencing and a sample pretreatment method for nucleic acid sequencing to avoid low sequencing accuracy caused by a low utilization rate of long nucleic acid fragments in nucleic acids during nanopore sequencing in the related art.

An embodiment of the present application provides a sample pretreatment device for nucleic acid sequencing.

The sample pretreatment device for nucleic acid sequencing includes a carrier, a first electrode, a second electrode, and a controller.

The carrier is provided with a pretreatment chamber. The carrier is also provided with a fluid inlet and a fluid outlet that communicate with the pretreatment chamber.

The first electrode is disposed on the top wall of the pretreatment chamber.

The second electrode is disposed on the bottom wall of the pretreatment chamber.

The first electrode and the second electrode are electrically connected to the controller.

An embodiment of the present application provides a sample pretreatment method for nucleic acid sequencing. The method is performed by using the preceding sample pretreatment device for nucleic acid sequencing.

The method includes introducing a sample into the pretreatment chamber through the fluid inlet before performing nucleic acid sequencing on the sample; and applying an alternating current through the controller to the first electrode and the second electrode to separate a first nucleic acid fragment and a second nucleic acid fragment to make the second nucleic acid fragment move towards the upper layer of a fluid, and the first nucleic acid fragment move towards the lower layer of the fluid.

### BRIEF DESCRIPTION OF DRAWINGS

FIG. 1 is a view of a sample pretreatment device for nucleic acid sequencing according to an embodiment.
FIG. 2 is a sectional view of a sample pretreatment device for nucleic acid sequencing according to an embodiment.
FIG. 3 is an enlarged view of part A of FIG. 2.
FIG. 4 is a first exploded view of a sample pretreatment device for nucleic acid sequencing according to an embodiment.
FIG. 5 is a second exploded view of a sample pretreatment device for nucleic acid sequencing according to an embodiment.
FIG. 6 is a view of a sample pretreatment device for nucleic acid sequencing in an initial state according to an embodiment.
FIG. 7 is a view of a sample pretreatment device for nucleic acid sequencing including a pre-filled solution according to an embodiment.
FIG. 8 is a first view of a sample pretreatment device for nucleic acid sequencing with a loaded base solution according to an embodiment.
FIG. 9 is a second view of a sample pretreatment device for nucleic acid sequencing with a loaded base solution according to an embodiment.
FIG. 10 is a third view of a sample pretreatment device for nucleic acid sequencing with a loaded base solution according to an embodiment.
FIG. 11 is a view of a sample pretreatment device for nucleic acid sequencing containing a base solution after fluid displacement according to an embodiment.
FIG. 12 is a view of a sample pretreatment device for nucleic acid sequencing with a sample introduced according to an embodiment.
FIG. 13 is a view of a sample pretreatment device for nucleic acid sequencing with a sample settled according to an embodiment.

### Reference list

100. sample; 200. pre-filled solution; 300. base solution;
10. carrier; 101. pretreatment chamber; 1011. guiding bevel; 102. fluid inlet; 103. fluid outlet; 104. sequencing chamber; 105. aperture array; 106. waste reservoir; 107. sample exchange channel; 1071. fluid exchange port; 1072. straight channel; 1073. waste port; 108. sample loading channel; 109. gas guiding channel;
11. upper cover plate; 111. first protrusion; 112. first groove; 12. middle plate; 121. first mounting groove; 122. first connection hole; 123. second mounting groove; 13. bottom plate; 131. second connection hole; 132. third connection hole;
20. first electrode;
30. second electrode;
40. sealing gasket

### DETAILED DESCRIPTION

In the description of the present application, the terms "joined", "connected", and "fixed" are to be understood in a broad sense unless otherwise expressly specified and limited. For example, the term "connected" may refer to "fixedly connected", "detachably connected", or "integrated", may refer to "mechanically connected" or "electrically connected", may refer to "connected directly" or "connected indirectly through an intermediary", or may refer to "connected inside two elements" or "an interaction relation between two elements". For those of ordinary skill in the art, specific meanings of the preceding terms in the present application may be understood based on specific situations.

In the present application, unless otherwise expressly specified and limited, when a first feature is described as being "on" or "below" a second feature, the first feature and the second feature may be in direct contact or be in contact via another feature between the two features instead of being in direct contact. Moreover, when the first feature is described as "on", "above", or "over" the second feature, the first feature is right on, above, or over the second feature, the first feature is obliquely on, above, or over the second feature, or the first feature is simply at a higher level than the second feature. When the first feature is described as "under", "below", or "underneath" the second feature, the first feature is right under, below, or underneath the second feature, the first feature is obliquely under, below, or underneath the second feature, or the first feature is simply at a lower level than the second feature.

The solutions of the present application are described below in conjunction with the drawings and embodiments.

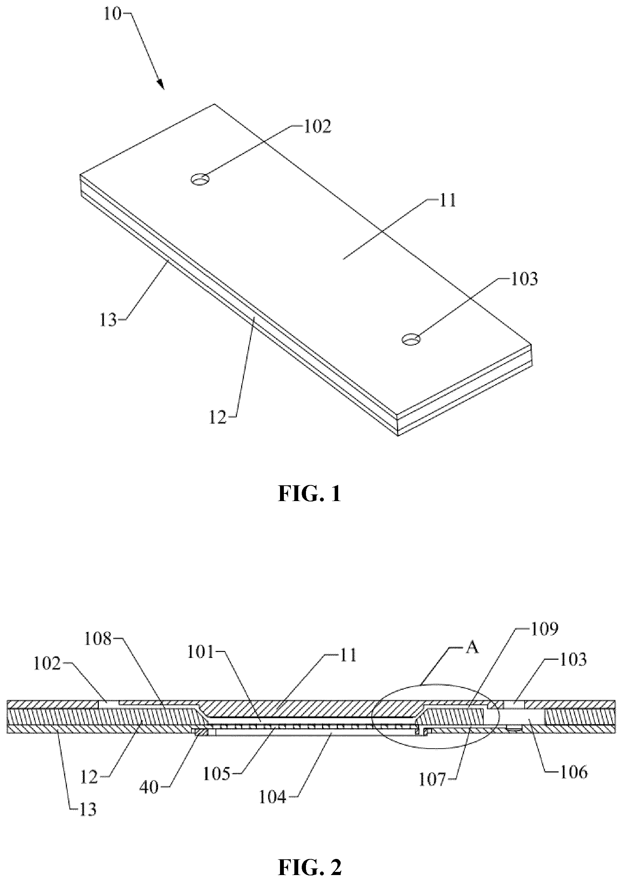

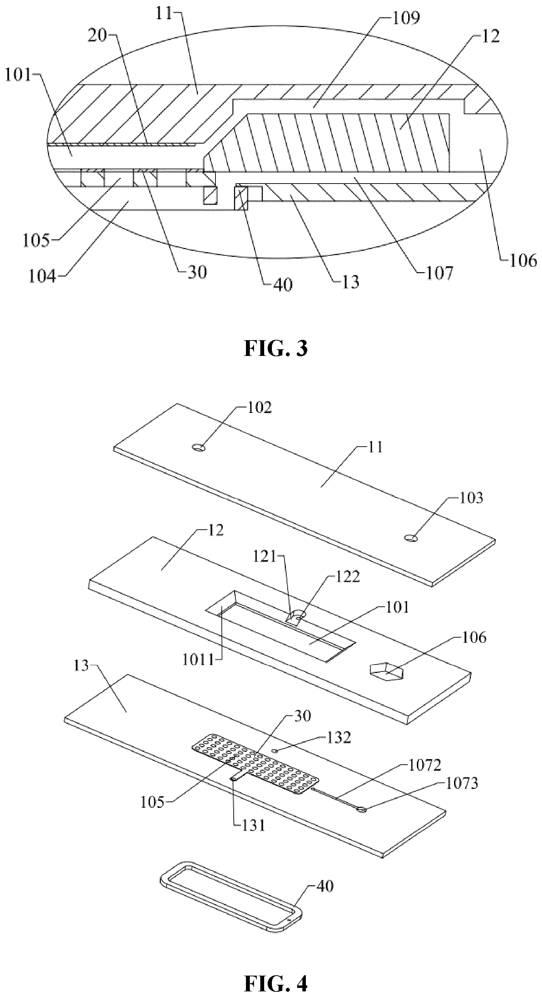

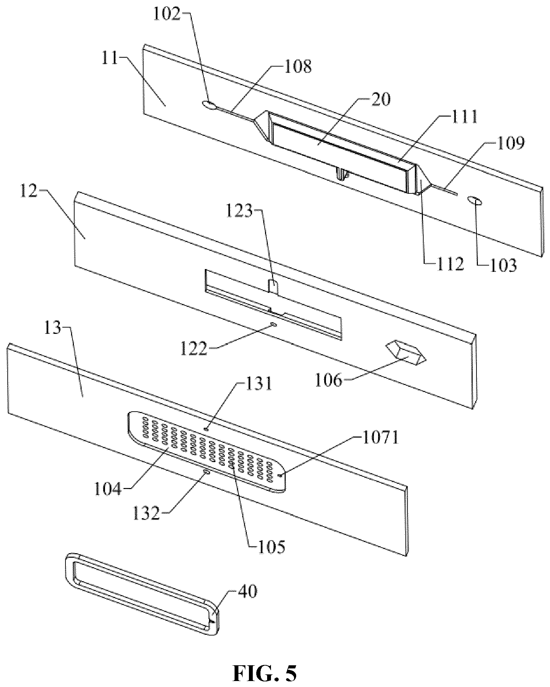

Referring to FIG. 1 to FIG. 5, an embodiment provides a sample pretreatment device for nucleic acid sequencing. With this device, a sample 100 is pretreated before the nucleic acid sequencing, resulting in the separation of first nucleic acid fragments and second nucleic acid fragments in the sample 100. The sample 100 of the embodiment is a nucleic acid sample under test. The first nucleic acid fragments may be long nucleic acid fragments and the second nucleic acid fragments may be short nucleic acid fragments. For example, the threshold for distinguishing the short nucleic acid fragments and the long nucleic acid fragments may be within a range of 1 - 4 kb (kilobase pairs). Generally, nucleic acid fragments with a length less than 1 kb are regarded as the short nucleic acid fragments, and nucleic acid fragments with a length greater than 1 kb are regarded as the long nucleic acid fragments; or nucleic acid fragments with a length less than 4 kb are regarded as the short nucleic acid fragments, and nucleic acid fragments with a length greater than 4 kb are regarded as the long nucleic acid fragments.

For example, the sample pretreatment device for nucleic acid sequencing includes a carrier 10, a first electrode 20, a second electrode 30, and a controller. The carrier 10 is provided with a pretreatment chamber 101. The carrier 10 is also provided with a fluid inlet 102 and a fluid outlet 103 that communicate with the pretreatment chamber 101. The first electrode 20 is disposed on the top wall of the pretreatment chamber 101, and the second electrode 30 is disposed on the bottom wall of the pretreatment chamber 101. The first electrode 20 and the second electrode 30 are electrically connected to the controller. The controller can adjust the frequency and potential difference of an alternating current between the first electrode 20 and the second electrode 30. The structures and working principles of the controller, the first electrode 20, and the second electrode 30 are known in the related art and thus are not described here.

Before the nucleic acid sequencing, the sample 100 is introduced into the pretreatment chamber 101 through the fluid inlet 102, and the alternating current is applied to the first electrode 20 and the second electrode 30 through the controller to separate the long nucleic acid fragments and the short nucleic acid fragments, thereby the short nucleic acid fragments migrate towards the upper layer of the fluid and the long nucleic acid fragments migrate towards the lower layer of the fluid. During a formal sequencing process, the long nucleic acid fragments in the lower layer are closer to the nanopore aperture array and thus have a higher electrophoretic priority, enabling faster translocation through the nanopore aperture array, thereby improving the utilization rate of the sample 100 and enhancing the accuracy of sequencing results.

The first electrode 20 and the second electrode 30 are arranged to form a pair of separated electrodes and a stable dielectrophoretic field can be generated between the pair of separated electrodes. Nucleic acid fragments with different charges are gradually pre-separated under the dielectrophoretic field so that the short nucleic acid fragments move towards the upper layer of the fluid and the long nucleic acid fragments move towards the lower layer of the fluid. The nanopore aperture array is disposed below the pretreatment chamber 101 according to the downward flowing characteristic of the fluid. In this way, the long nucleic acid fragments are closer to the nanopore aperture array and thus are granted the higher electrophoretic priority, thereby improving the utilization rate of the sample 100.

For example, the carrier 10 is also provided with a sequencing chamber 104. The sequencing chamber 104 is positioned below the pretreatment chamber 101 and communicates with the pretreatment chamber 101 through an aperture array 105. When the sample 100 in the pretreatment chamber 101 is settled into the sequencing chamber 104 through the aperture array 105, the long nucleic acid fragments in the lower layer of the fluid are closer to the aperture array 105 and thus are granted the higher electrophoretic priority, thereby improving the utilization rate of the sample 100. The aperture array 105 includes multiple nanopores arranged in an array.

It is to be understood that during the nucleic acid sequencing, the sample 100 is required to pass through the aperture array 105 and then enters a gene sequencing device after passing through the aperture array 105. The gene sequencing device may be a conventional sequencing device. In the embodiment, the sequencing chamber 104 is connected to a sequencing chip of the gene sequencing device and the sample 100 entering the sequencing chamber 104 is able to contact the sequencing chip to complete sequencing.

The carrier 10 is also provided with a waste reservoir 106. The waste reservoir 106 communicates with the sequencing chamber 104 and the fluid outlet 103 separately. Fluid displacement is required in the process of introducing the sample 100 into the pretreatment chamber 101 through the fluid inlet 102. For example, in an initial state, a pre-filled solution 200 is contained in the pretreatment chamber 101 and the sequencing chamber 104. The pre-filled solution 200 is displaced with a base solution 300, and then the sample 100 is loaded into the pretreatment chamber 101. The sample 100 can float on the base solution 300. After the pre-separation of the sample 100 is completed, the base solution 300 is aspirated so that the pre-separated sample 100 can flow downward into the sequencing chamber 104. It is to be understood that the sample 100 and the base solution 300 are immiscible, so the sample 100 and the base solution 300 are layered. The sample 100, lower in density, stays in the upper layer while the base solution 300 stays in the lower layer.

The pre-filled solution 200 is displaced with the base solution 300 as follows: the base solution 300 is loaded through the fluid inlet 102 by using a pipette, and the base solution 300 floats on the pre-filled solution 200; then the waste liquid is aspirated from the waste reservoir 106 through the fluid outlet 103 to make the pre-filled solution 200 in the pretreatment chamber 101 gradually decrease until the base solution 300 fills the sequencing chamber 104 and is contained partially or fully in the pretreatment chamber 101. The sample 100 is loaded into the pretreatment chamber 101 as follows: the sample 100 is loaded through the fluid inlet 102 by using the pipette, the sample 100 floats on the base solution 300, so that part of the base solution 300 is squeezed into the waste reservoir 106; and then the fluid inlet 102 and the fluid outlet 103 are closed. After the pre-separation is completed, the base solution 300 is aspirated as follows: the fluid inlet 102 and the fluid outlet 103 are opened and the waste liquid is aspirated from the waste reservoir 106 through the fluid outlet 103 to make the base solution 300 in the pretreatment chamber 101 decrease, and the pre-separated sample 100 flows downward into the sequencing chamber 104 through the aperture array 105.

The pre-filled solution 200 and the base solution 300 may each be a buffer solution in the related art and play a protective role. The base solution 300 is used for displacing the pre-filled solution 200 before pretreating the sample 100 and is used for ensuring the floating position of the sample 100 in the pretreatment process.

The waste reservoir 106 is disposed in alignment with the fluid outlet 103 so that the pipette can easily pipette the waste liquid from the waste reservoir 106 through the fluid outlet 103. The waste reservoir 106 communicates with the sequencing chamber 104 through a sample exchange channel 107, facilitating the smooth flow of fluid from the sequencing chamber 104 into the waste reservoir 106. In the embodiment, the fluid inlet 102 and the fluid outlet 103 are positioned on two opposite sides of the pretreatment chamber 101, so that the waste reservoir 106 and the fluid inlet 102 are positioned on the two opposite sides of the pretreatment chamber 101.

The carrier 10 is also provided with a sample loading channel 108. The pretreatment chamber 101 is connected to the fluid inlet 102 through the sample loading channel 108. The sample loading channel 108 is configured to load the fluid. In the embodiment, a guiding bevel 1011 is provided at the edge of the pretreatment chamber 101, and the end of the sample loading channel 108 extends to the guiding bevel 1011 to direct the fluid to smoothly enter the pretreatment chamber 101. The guiding bevel 1011 may be inclined at 60 degrees.

The carrier 10 is also provided with a gas guiding channel 109. One end of the gas guiding channel 109 communicates with the pretreatment chamber 101 and the other end of the gas guiding channel 109 communicates with the fluid outlet 103. In the process of the fluid flowing into the pretreatment chamber 101, gas in the pretreatment chamber 101 passes through the gas guiding channel 109 and is discharged from the fluid outlet 103, thereby ensuring the pressure balance in the pretreatment chamber 101. The device structure is simplified since the gas and the fluid share the fluid outlet 103. It is to be understood that the gas guiding channel 109 is positioned above the sample exchange channel 107 to prevent the fluid from entering the gas guiding channel 109. The fluid inlet 102 is for sample loading, and the fluid outlet 103 is for aspirating the waste liquid, which are both for balancing pressure.

In the embodiment, the carrier 10 is composed of several detachable parts, facilitating processing, production, and assembly of electrodes. For example, the carrier 10 includes an upper cover plate 11, a middle plate 12, and a bottom plate 13 connected in sequence from top to bottom. The middle plate 12 is provided with the pretreatment chamber 101. The upper cover plate 11 is provided with the fluid inlet 102 and the fluid outlet 103.

The sample loading channel 108, the gas guiding channel 109, and the first electrode 20 are all provided on the upper cover plate 11 and positioned on a side of the upper cover plate 11 facing the middle plate 12. For example, the upper cover plate 11 is provided with a first protrusion 111 and a first groove 112 around the first protrusion 111, the first electrode 20 is disposed on the first protrusion 111, and the sample loading channel 108 and the gas guiding channel 109 both communicate with the first groove 112.

The waste reservoir 106 is provided on the middle plate 12. Both the pretreatment chamber 101 and the waste reservoir 106 extend through the thickness of the middle plate 12. The gas guiding channel 109 may directly communicate with the fluid outlet 103 or may indirectly communicate with the fluid outlet 103. In the embodiment, the gas guiding channel 109 communicates with the fluid outlet 103 through the waste reservoir 106.

The middle plate 12 is provided with a first mounting groove 121. An electrode lead of the first electrode 20 is positioned within the first mounting groove 121. For example, the first mounting groove 121 defines a first connection hole 122 through which the electrode lead of the first electrode 20 extends. The middle plate 12 is also provided with a second mounting groove 123. An electrode lead of the second electrode 30 is positioned within the second mounting groove 123. The first mounting groove 121 and the second mounting groove 123 are distributed on two opposite sides of the middle plate 12.

The bottom plate 13 defines the aperture array 105. The aperture array 105 is aligned with the pretreatment chamber 101, thereby the top surface of the bottom plate 13 serves as the bottom wall of the pretreatment chamber 101. It is to be understood that the second electrode 30 does not affect fluid flow through the aperture array 105.

For example, the bottom plate 13 defines a second connection hole 131 in communication with the second mounting groove 123, and the electrode lead of the second electrode 30 extends through the second connection hole 131. The bottom plate 13 also defines a third connection hole 132 aligned with the first connection hole 122, so that the electrode lead of the first electrode 20 also extends through the third connection hole 132.

The sequencing chamber 104 is provided on the bottom plate 13 and positioned on a side of the bottom plate 13 facing away from the middle plate 12. The sequencing chamber 104 is disposed in alignment with the aperture array 105. A sealing gasket 40 is disposed at the edge of the sequencing chamber 104. The sealing gasket 40 seals the sequencing chamber 104 when the sequencing chamber 104 is connected to the gene sequencing device.

The sample exchange channel 107 includes a fluid exchange port 1071, a straight channel 1072, and a waste port 1073 that communicate in sequence. The straight channel 1072 is provided on the top surface of the bottom plate 13. The fluid exchange port 1071 extends through the bottom plate 13 to connect the sequencing chamber 104 to the straight channel 1072. The waste port 1073 is positioned within the waste reservoir 106. The fluid in the sequencing chamber 104 is able to flow into the waste reservoir 106 along the fluid exchange port 1071, the straight channel 1072, and the waste port 1073.

The first electrode 20 and the second electrode 30 may be prepared by common techniques such as vapor deposition, electroplating, or screen printing. An electrical connection can be achieved by contact between the first electrode 20 and a pin. Copper is deposited in the second connection hole 131 to serve as an electrical connection contact of the second electrode 30. The upper cover plate 11, the middle plate 12, and the bottom plate 13 can be assembled by a sealing process such as hot pressing, laser welding, or ultrasonic welding. The sealing gasket 40 is made of a flexible material such as silica gel or rubber.

In the embodiment, the upper cover plate 11 is made of transparent high impact polystyrene (HIPS), the first electrode 20 and the second electrode 30 are made of platinum, the middle plate 12 is made of black HIPS, and the bottom plate 13 is made of transparent HIPS. The fluid inlet 102 and the fluid outlet 103 are each a circular port. The sample loading channel 108 and the gas guiding channel 109 have the same width and the same depth. The first protrusion 111 is a boss inclined at 45°. The waste reservoir 106 is in the shape of a regular hexagon. Multiple components may be set according to actual requirements in terms of size.

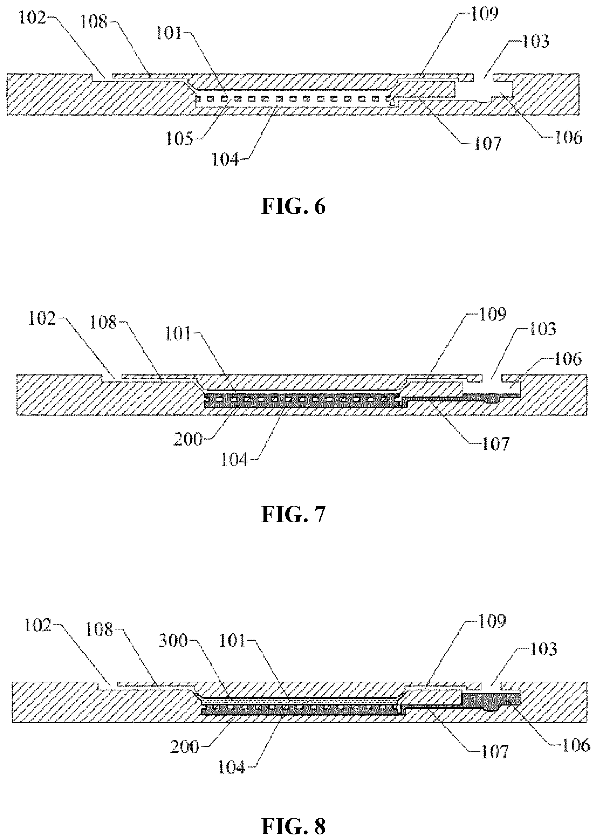

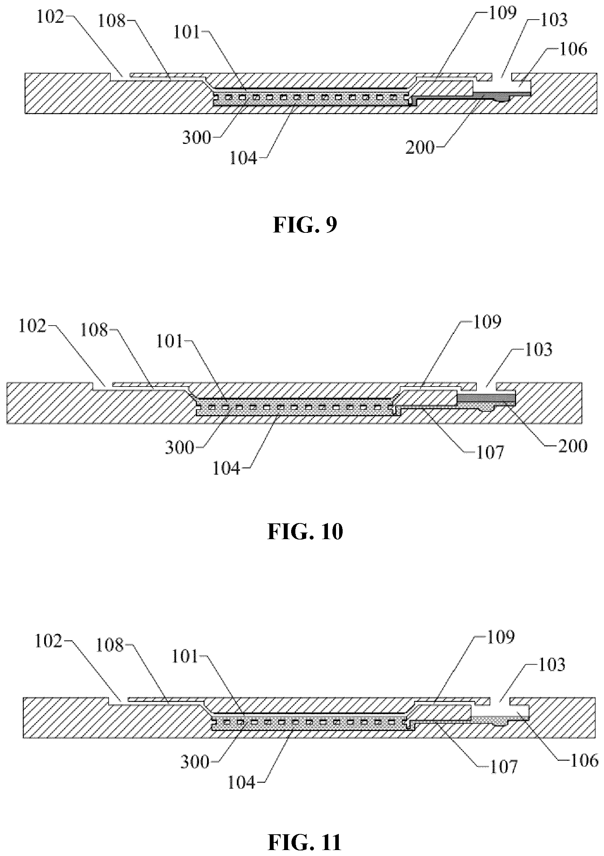

Referring to FIG. 6 to FIG. 13, the embodiment provides a sample pretreatment method for nucleic acid sequencing. The method is performed by using the preceding sample pretreatment device for nucleic acid sequencing.

The method includes introducing the sample 100 into the pretreatment chamber 101 through the fluid inlet 102 before performing the nucleic acid sequencing on the sample 100; and applying the alternating current by the controller to the first electrode 20 and the second electrode 30 to separate the long nucleic acid fragment and the short nucleic acid fragment to make the short nucleic acid fragment move towards the upper layer of the fluid and the long nucleic acid fragment move towards the lower layer of the fluid.

It is to be understood that before using the sample pretreatment device for nucleic acid sequencing, the sample pretreatment device for nucleic acid sequencing is required to be assembled and connected to a matched gene sequencing device to enable the sequencing chamber 104 to be selectively connected to the gene sequencing device.

Introducing the sample 100 into the pretreatment chamber 101 through the fluid inlet 102 includes displacing the pre-filled solution 200 and loading the sample 100. Displacing the pre-filled solution 200 includes that the pre-filled solution 200 in the pretreatment chamber 101 is displaced with the base solution 300; loading the sample 100 includes that: the sample 100 is loaded through the fluid inlet 102, the sample 100 floats on the base solution 300 so that part of the base solution 300 is squeezed into the waste reservoir 106; and then the fluid inlet 102 and the fluid outlet 103 are closed.

As shown in FIG. 7, in the initial state, the pre-filled solution 200 is contained in the pretreatment chamber 101 and the sequencing chamber 104. It is to be understood that part of the pre-filled solution 200 flows into the waste reservoir 106 since the sequencing chamber 104 communicates with the waste reservoir 106.

As shown in FIG. 8 to FIG. 11, displacing the pre-filled solution 200 with the base solution 300 includes that: the base solution 300 is loaded through the fluid inlet 102 by using the pipette, the base solution 300 floats on the pre-filled solution 200, and the pre-filled solution 200 flows towards the waste reservoir 106; then the waste liquid is pipetted from the waste reservoir 106 through the fluid outlet 103 by using the pipette to make the pre-filled solution 200 in the pretreatment chamber 101 and the sequencing chamber 104 decreaseuntil the base solution 300 fills the sequencing chamber 104 and is contained partially or fully in the pretreatment chamber 101. It is to be understood that part of the base solution 300 also flows into the waste reservoir 106 since the sequencing chamber 104 communicates with the waste reservoir 106. Thus, the pre-filled solution 200 has been displaced.

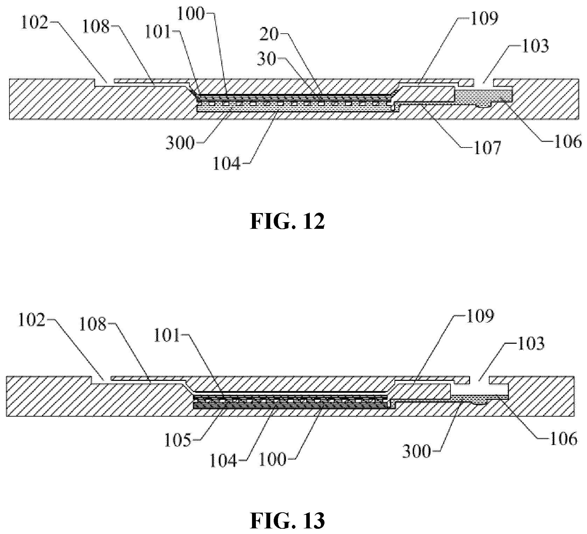

As shown in FIG. 12, in the process of introducing the sample 100, the sample 100 is loaded through the fluid inlet 102 by using the pipette, the sample 100 floats on the base solution 300, and the base solution 300 flows towards the waste reservoir 106; then the waste liquid is pipetted from the waste reservoir 106 through the fluid outlet 103 by using the pipette to make the base solution 300 in the pretreatment chamber 101 decrease until the sample 100 is not lower than the bottom wall of the pretreatment chamber 101.

Next, the pre-separation is performed. The alternating current is applied to the first electrode 20 and the second electrode 30 by the controller to separate the long nucleic acid fragments and the short nucleic acid fragments, thus the short nucleic acid fragments move towards the upper layer of the fluid and the long nucleic acid fragments move towards the lower layer of the fluid. The alternating current may be configured with a first set frequency and a first set potential difference, and may be applied for a first set duration.

After the pre-separation is completed, the fluid inlet 102 and the fluid outlet 103 are opened, and the waste liquid is aspirated from the waste reservoir 106 through the fluid outlet 103 to make the base solution 300 in the sequencing chamber 104 decrease. The pre-separated sample 100 flows downward (or settles) into the sequencing chamber 104 through the aperture array 105. When the sequencing chamber 104 is opened, the pre-separated sample 100 can enter the gene sequencing device to achieve gene sequencing.

In the embodiment, in the initial state, 100 microliters of pre-filled solution 200 is loaded into the sample pretreatment device for nucleic acid sequencing to play the protective role, and the fluid inlet 102 and the fluid outlet 103 are closed by rubber plugs, respectively. The bottom plate 13 and the sequencing chip are assembled and placed into the gene sequencing device, and instrument preparation is completed. The rubber plug at the fluid inlet 102 and the rubber plug at the fluid outlet 103 are removed to make the fluid inlet 102 and the fluid outlet 103 communicate with the ambient air.

When displacing the pre-filled solution 200, 80 microliters of base solution 300 is loaded through the fluid inlet 102 by using the pipette, and then 80 microliters of waste liquid is pipetted from the bottom of the waste reservoir 106 through the fluid outlet 103. This operation is repeated once to displace the pre-filled solution 200 with the base solution 300.

When loading the sample 100, 80 microliters of sample 100 is loaded through the fluid inlet 102 by using the pipette, and then the fluid inlet 102 and the fluid outlet 103 are closed.

During the pre-separation, an alternating current configured with a frequency of 10 MHz and a potential difference of 10 V is applied to the first electrode 20 and the second electrode 30 by the controller for 3 min. Thus, the long nucleic acid fragments and the short nucleic acid fragments are separated.

Next, the fluid inlet 102 and the fluid outlet 103 are opened, 80 microliters of waste liquid is pipetted from the bottom of the waste reservoir 106 through the fluid outlet 103 by using the pipette, the pre-separated sample 100 flows downward, and most of the remaining base solution 300 flows into the waste reservoir 106 through the sample exchange channel 107.

In summary, the sample pretreatment device and method for nucleic acid sequencing of the embodiments achieve the pre-separation of the sample 100 by using the alternating current dielectrophoresis technology and the electrode pair, and achieve the displacement of the pre-filled solution 200 with the base solution 300 by using the laminar flow technology and the communicating structure. Additionally, the manual operation of the pipettes can be replaced by a liquid path of automated loading and/or washing and a control system.

## Claims

1. A sample pretreatment device for nucleic acid sequencing, comprising:
a carrier (10) provided with a pretreatment chamber (101), a fluid inlet (102) and a fluid outlet (103), wherein the fluid inlet (102) and the fluid outlet (103) communicate with the pretreatment chamber (101);
a first electrode (20) disposed on a top wall of the pretreatment chamber (101);
a second electrode (30) disposed on a bottom wall of the pretreatment chamber (101); and
a controller electrically connected to the first electrode (20) and the second electrode (30).

2. The device of claim 1, wherein the carrier (10) is further provided with a sequencing chamber (104) which is positioned below the pretreatment chamber (101) and communicates with the pretreatment chamber (101) through an aperture array (105).

3. The device of claim 2, wherein the carrier (10) is further provided with a waste reservoir (106) which communicates with the sequencing chamber (104) and the fluid outlet (103).

4. The device of claim 3, wherein the waste reservoir (106) is disposed in alignment with the fluid outlet (103).

5. The device of claim 3, wherein the waste reservoir (106) communicates with the sequencing chamber (104) through a sample exchange channel (107).

6. The device of claim 1, wherein the carrier (10) is further provided with a sample loading channel (108), wherein the fluid inlet (102) communicates with the pretreatment chamber (101) through the sample loading channel (108).

7. The device of claim 6, wherein an edge of the pretreatment chamber (101) is provided with a guiding bevel (1011), and an end of the sample loading channel (108) extends to the guiding bevel (1011).

8. The device of claim 1, wherein the carrier (10) is further provided with a gas guiding channel (109), a first end of the gas guiding channel (109) communicates with the pretreatment chamber (101), and a second end of the gas guiding channel (109) communicates with the fluid outlet (103).

9. The device of any one of claims 1 to 8, wherein the carrier (10) comprises an upper cover plate (11), a middle plate (12), and a bottom plate (13) connected in sequence from top to bottom, the middle plate (12) is provided with the pretreatment chamber (101), and the upper cover plate (11) is provided with the fluid inlet (102) and the fluid outlet (103).

10. A sample pretreatment method for nucleic acid sequencing, using the sample pretreatment device for nucleic acid sequencing of any one of claims 1 to 9 and comprising:
introducing a sample into the pretreatment chamber through the fluid inlet before performing nucleic acid sequencing on the sample; and
applying, by the controller, an alternating current to the first electrode and the second electrode to separate a first nucleic acid fragment and a second nucleic acid fragment to make the second nucleic acid fragment move towards an upper layer of a fluid and the first nucleic acid fragment move towards a lower layer of the fluid.
